(19) Europäisches Patentamt
European Patent Office
Office européen des brevets

(11) Veröffentlichungsnummer: **0 516 015 A1**

# (12) EUROPÄISCHE PATENTANMELDUNG

(21) Anmeldenummer: **92108785.4**

(22) Anmeldetag: **25.05.92**

(51) Int. Cl.5: **C07D 313/00**, A61K 31/365, C07D 493/04, C07H 17/08, C12P 17/08, //(C07D493/04, 313:00,303:00),(C07D493/04, 317:00,313:00)

(30) Priorität: **31.05.91 DE 4117824**

(43) Veröffentlichungstag der Anmeldung: **02.12.92 Patentblatt 92/49**

(84) Benannte Vertragsstaaten: **AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Granzer, Ernold, Dr.**
**Falkensteiner Strasse 24**
**W-6233 Kelkheim(DE)**
Erfinder: **Hammann, Peter, Dr.**
**Bürgermeister-Rühl-Strasse 20**
**W-6223 Babenhausen(DE)**
Erfinder: **Kirsch, Reinhard, Dr.**
**Johannesallee 18**
**W-6230 Frankfurt am Main 80(DE)**

## (54) Substituierte Decarestrictine, Verfahren zu ihrer Herstellung und Verwendung derselben.

(57) Die Erfindung betrifft substituierte Decarestrictine der Formel I

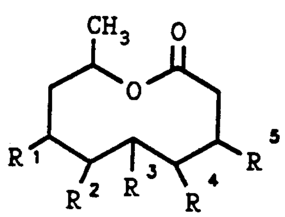

(I)

wobei $R^1$ Mono- oder Disaccharid, Hydroxy-, Formyl- oder Carbonyl-Gruppe, -O-Alkyl, -O-Alkenyl, -O-C(O)-Alkyl, -O-C(O)-Alkenyl, -O-C(O)-Phenyl, worin der Phenylrest substituiert sein kann, bedeutet, $R^2$ und $R^3$ Wasserstoff, eine Doppelbindung oder Oxiranring bedeuten, $R^4$ oder $R^5$ für Hydroxy- oder Carbonyl-Gruppe, -O-Alkyl, -O-Alkenyl, -O-C(O)-Alkyl, -O-C(O)-Alkenyl, -O-C(O)-Phenyl, worin der Phenylrest substituiert sein kann, bedeuten oder $R^4$ und $R^5$ zusammen ein Ketal bilden können, sowie deren physiologisch verträglichen Salze; Verfahren zur Herstellung dieser Decarestrictinderivate, sowie deren Verwendung als Arzneimittel mit antibakterieller, antiviraler und lipidsenkender Wirkung.

EP 0 516 015 A1

Die Erfindung betrifft substituierte Decarestrictine (Zehnringlactone), Verfahren zu ihrer Herstellung, sowie deren Verwendung, insbesondere als Arzneimittel, welche antivirale, antibakterielle und lipidregulatorische Eigenschaften besitzen.

Decarestrictine der Formeln Ia und Ib,

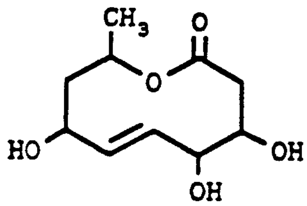

Ia

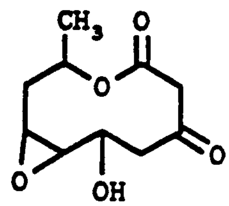

Ib

wurden bereits in EP-A-89 104 160.0 beschrieben. Sie zeigen antibakterielle Wirkung. Die Naturprodukte der Formeln Ia und Ib werden durch Kultivierung von Mikroorganismen der Spezies Penicillium spec. DSM 4209 und DSM 4210 in einem Nährmedium erhalten. In der Europäischen Patentanmeldung EP-A-90 122 907.0 werden Derivate ausgehend von der Verbindung Ib bereits vorgeschlagen, die die Cholesterin-Biosynthese beeinflussen.

In dem Bestreben weitere wirksame Decarestrictine zu finden, wurde nun gefunden, daß neue Derivate der Decarestrictine die Cholesterin-Biosynthese wirkungsvoll hemmen und antibakterielle und antivirale Eigenschaften besitzen.

Die Erfindung betrifft somit Decarestrictine der Formel I

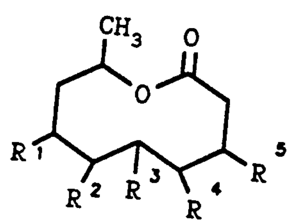

(I)

I

wobei $R^1$ für

a) Hydroxy,
b) Formyl,
c) Carbonyl,
d) -O-($C_1$-$C_{10}$)-Alkyl, worin die Kohlenstoffkette geradkettig oder verzweigt sein kann,
e) -O-($C_2$-$C_{10}$)-Alkenyl, worin die Kohlenstoffkette geradkettig oder verzweigt sein kann und eine oder mehrere Doppelbindungen enthält,
f) -O-C(O)-($C_1$-$C_{10}$)-Alkyl, worin die Kohlenstoffkette geradkettig oder verzweigt sein kann,
g) -O-C(O)-($C_2$-$C_{10}$)-Alkenyl, worin die Kohlenstoffkette geradkettig oder verzweigt sein kann und eine oder mehrere Doppelbindungen enthält,
h) -O-C(O)-Phenyl,
i) -O-C(O)-Phenyl, ein- bis dreifach substituiert am Phenyl-Rest durch
   1) Halogen, wie Fluor, Chlor oder Brom,
   2) ($C_1$-$C_8$)-Alkyl, worin die Kohlenstoffkette geradkettig oder verzweigt sein kann,
   3) $NO_2$,
   4) CN,
   5) $CF_3$,
   6) Phenyl oder
   7) Phenoxy,

j) Mono- oder Disaccharid oder
k) Mono- oder Disaccharid, deren Hydroxylgruppen mit Schutzgruppen geschützt sind, steht,
$R^2$ und $R^3$ für Wasserstoff stehen oder
$R^2$ und $R^3$ zusammen eine Doppelbindung oder einen Oxiranring bilden,
$R^4$ oder $R^5$ unabhängig voneinander für
a) Hydroxy,
b) Carbonyl,
c) -O-($C_1$-$C_{10}$)-Alkyl, worin die Kohlenstoffkette geradkettig oder verzweigt sein kann,
d) -O-($C_2$-$C_{10}$)-Alkenyl, worin die Kohlenstoffkette geradkettig oder verzweigt sein kann und eine oder mehrere Doppelbindungen enthält.
e) -O-C(O)-($C_1$-$C_{10}$)-Alkyl, worin die Kohlenstoffkette geradkettig oder verzweigt sein kann,
f) -O-C(O)-($C_2$-$C_{10}$)-Alkenyl, worin die Kohlenstoffkette geradkettig oder verzweigt sein kann und eine oder mehrere Doppelbindungen enthält,
g) -O-C(O)-Phenyl oder
h) -O-C(O)-Phenyl, ein- bis dreifach substituiert am Phenyl-Rest durch
1) Halogen, wie Fluor, Chlor oder Brom,
2) ($C_1$-$C_8$)-Alkyl, worin die Kohlenstoffkette geradkettig oder verzweigt sein kann,
3) $NO_2$,
4) CN,
5) $CF_3$,
6) Phenyl oder
7) Phenoxy steht, oder
$R^4$ und $R^5$ zusammen einen Rest der Formel II oder III

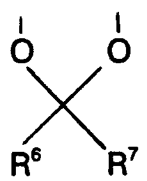 (II)

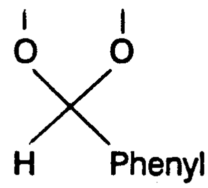 (III)

bilden, wobei $R^6$ oder $R^7$ unabhängig voneinander
a) Wasserstoff oder
b) ($C_1$-$C_8$)-Alkyl, worin die Kohlenstoffkette geradkettig oder verzweigt sein kann, bedeuten,
sowie deren physiologisch verträglichen Salze,
ausgenommen die Verbindung der Formel I, wobei $R^1$ und $R^4$ für Hydroxylgruppe, $R^5$ für Hydroxyl- oder Carbonylgruppe steht und $R^2$ und $R^3$ zusammen eine Doppelbindung bilden.

Insbesondere betrifft die Erfindung Decarestrictine der Formel I wobei $R^1$ für
a) Hydroxy,
b) Formyl,
c) Carbonyl,
d) -O-($C_1$-$C_4$)-Alkyl,
e) -O-C(O)-($C_1$-$C_4$)-Alkyl, worin die Kohlensotffkette geradkettig oder verzweigt sein kann,
f) -O-C(O)-($C_2$-$C_4$)-Alkenyl, worin die Kohlenstoffkette geradkettig oder verzweigt sein kann,
g) -O-C(O)-Phenyl,
h) -O-C(O)-Phenyl, ein- bis dreifach substituiert am Phenyl-Rest durch Brom,
steht oder
i) Monosaccharid, dessen Hydroxylgruppen durch Benzoyl geschützt sind,
$R^2$ und $R^3$ für Wasserstoff stehen oder
$R^2$ und $R^3$ zusammen eine Doppelbindung oder einen Oxiranring bilden,
$R^4$ oder $R^5$ unabhängig voneinander
a) Hydroxy,
b) Carbonyl,
c) -O-($C_1$-$C_4$)-Alkyl,
d) -O-C(O)-($C_1$-$C_4$)-Alkyl, worin die Kohlenstoffkette geradkettig oder verzweigt sein kann,

e) -O-C(O)-($C_2$-$C_4$)-Alkenyl, worin die Kohlenstoffkette geradkettig oder verzweigt sein kann, oder

f) -O-C(O)-Phenyl bedeuten oder

$R^4$ oder $R^5$ zusammen einen Rest der Formel II oder III bilden, wobei $R^6$ oder $R^7$ für jeweils eine Methylgruppe steht, sowie deren physiologisch verträglichen Salze, ausgenommen die Verbindungen der Formel I, wobei $R^1$ und $R^4$ für Hydroxylgruppe, $R^5$ für Hydroxyl- oder Carbonylgruppe steht und $R^2$ und $R^3$ zusammen eine Doppelbindung bilden.

Besonders bevorzugt sind 4,5-Dihydroxy-O-isopropyliden-10-methyl-1-oxa-8-(2,3,4,6-tetra-O-butyl-α-D-glucopyranosyl-oxy)-2-cyclodecanon, 10-Methyl-1-oxa-4,5,8-tris-(n-butylcarbonyl-oxy)-6-cyclodecen-2-on, 10-Methyl-4,5,8-trimethoxy-1-oxa-6-cyclodecen-2-on, 8-Acetyloxy-4,5-dihydroxy-10-methyl-1-oxa-cyclodecan-2-on, 2-Carbonyl-10-methyl-1-oxa-4,5,8-trihydroxy-6-cyclodecenoxid, 10-Methyl-1-oxa-4,5,8-triacetyloxy-6-cyclodecen-2-on, 8-(2-Bromphenyl-carbonyloxy)-4,5-dihydroxy-O-isopropyliden-10-methyl-1-oxacyclodecan-2-on, 4,5-Dihydroxy-8-formyl-oxy-10-methyl-1-oxa-6-cyclodecen-2-on,4,8-Di-prop-1-en-1-yl-carbonyloxy-5-hydroxy-10-methyl-1-oxa-6-cyclodecen-2-on, 4,5-Dihydroxy-O-isopropyliden-8-methoxy-10-methyl-1-oxa-6-cyclodecan-2-on, 8-Butylcarbonyl-4,5-dihydroxy-O-isopropyliden-10-methyl-1-oxa-cyclodec an-2-on, 10-Methyl-1-oxa-4,5,8-trimethoxy-cyclodecan-2-on, 10-Methyl-1-oxa-4,5,8-tris-(acetyloxy)-cyclodecan-2-on, 10-Methyl-4,5,8-tris-(1-isopropen-2-yl-carbonyloxy)-1-oxa-6-cyclodecen-2-on, 10-Methyl-1-oxa-4,5,8-trihydroxy-cyclodecan-2-on, 10-Methyl-5,8-dimethoxy-4-hydroxy-1-oxa-6-cyclodecen-2-on, 10-Methyl-4,5,8-trihydroxy-4,5-O-isopropyliden-1-oxa-cyclodecan-2-on, 10-Methyl-1-oxa-4,5,8-trihydroxy-4,5-O-benzyliden-6-cyclodecen-2-on, 10-Methyl-8-acetyloxy,4,5-dihydrory-1-oxa-6-cyclodecen-2-on, 5,8-Bis-(phenylcarbonyloxy)-4-hydroxy-10-methyl-1-oxa-6-cyclodecen-2-on, 10-Methyl-1-oxa-4,5,8-tris-(ethylcarbonyloxy)-6-cyclodecen-2-on, 4,5-Dihydroxy-10-methyl-1-oxa-8-phenylcarbonyloxy-6-cyclodecen-2-on, 10-Methyl-1-oxa-4,5,8-triethoxy-6-cyclodecen-2-on, 4-Hydroxy-10-methyl-1-oxa-6-cyclodecen-2,5,8-trion.

Bei den genannten Zuckerresten handelt es sich um Mono- und Disaccharide wie sie beispielsweise angegeben sind im "Biochemischen Taschenbuch", 2. Auflage, 1. Teil, Seiten 107-177, Springer-Verlag, Berlin, Göttingen, Heidelberg, 1964. Insbesondere handelt es sich um Hexopyranoside und Hexofuranoside; ganz besonders bevorzugt sind Glucose, Fructose, Allose, Altrose, Mannose, Lactose und Galaktose. Die in den genannten Zuckerresten vorhandenen OH-Gruppen können gegebenenfalls mit in der Zuckerchemie üblichen Schutzgruppen wie Acetyl, Benzoyl, Benzyl oder Acetonyl geschützt sein. Der Zuckerrest kann glykosidisch oder über einen Orthoester gebunden sein.

Weiterhin betrifft die Erfindung ein Verfahren zur Herstellung von Decarestrictinen der Formel I, wobei man

a) die Verbindung der Formel Ia

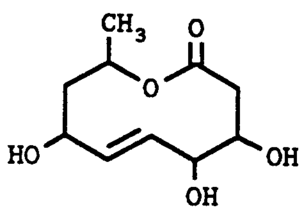

(Ia)

umsetzt mit einer Verbindung der Formel IV

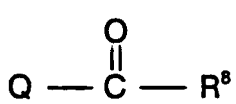

(IV)

wobei Q für

1) Hydroxy,

2) Halogen, wie Chlor oder Brom,

3) Imidazolid oder

4) Säureanhydrid steht,

$R^8$ für eine Alkyl- oder Alkenylgruppe mit 1 bis 10 C-Atomen steht, oder daß man

b) die Verbindung der Formel Ia mit einem geeigneten Enzym und einem Benzoyl- oder Acyldonor umsetzt,

oder daß man

c) die Verbindung der Formel Ia zu einer Verbindung der Formel V oxidiert,

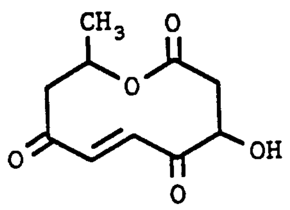

(V)

oder daß man

d) die Verbindung der Formel Ia an der Doppelbindung zu einer Verbindung der Formel VI epoxidiert,

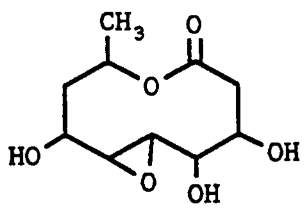

(VI)

oder daß man

e) die Verbindung der Formel Ia an der Doppelbindung zu einer Verbindung der Formel VII hydriert,

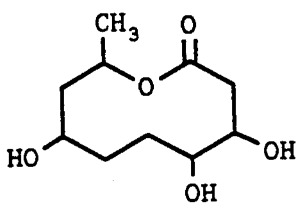

(VII)

oder daß man

f) die Verbindung der Formel Ia mit einer Verbindung der Formel

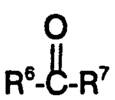

oder Benzaldehyd in das entsprechende Ketal der Formel VIII oder Benzylidenketal der Formel IX überführt,

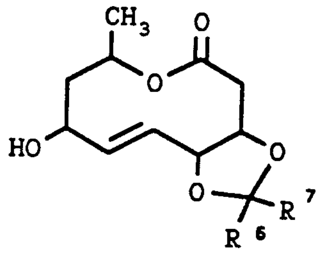

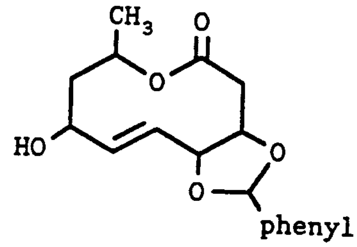

(VIII) (IX)

wobei $R^6$ oder $R^7$ unabhängig voneinander

1) Wasserstoff oder

2) ($C_1$-$C_8$)-Alkyl, worin die Kohlenstoffkette geradkettig oder verzweigt sein kann, bedeutet,

oder daß man

g) die Verbindung der Formel VIII oder IX an der Hydroxygruppe mit einer Verbindung der Formel X,

$R^{10}$-Hal (X)

wobei $R^{10}$ Mono- oder Disaccharid bedeutet und Hal für Fluor, Chlor, Brom oder Jod steht, umsetzt,

oder daß man

h) die Verbindung der Formel Ia mit einer Verbindung der Formel XI

$R^9$ - Abg (XI)

umsetzt, wobei $R^9$ für

1) ($C_1$-$C_{10}$)-Alkyl, worin die Kohlenstoffkette geradkettig oder verzweigt sein kann, oder

2) ($C_2$-$C_{10}$)-Alkenyl, worin die Kohlenstoffkette geradkettig oder verzweigt sein kann und eine oder mehrere Doppelbindungen enthält, steht und

Abg Chlor, Brom, Jod, Sulfat, Mesylat oder Tosylat bedeutet,

oder daß man

zwei oder mehrere der obengenannten Verfahrensvarianten a) bis h) hintereinander ausführt oder daß man die Verbindung der Formel I in ihre physiologisch verträglichen Salze überführt.

Die zur Herstellung von Verbindungen der Formel I notwendige Verbindung der Formel Ia kann beispielsweise nach dem in der europäischen Patentanmeldung EP-A-89 104 160 - auf die an dieser Stelle ausdrücklich Bezug genommen wird - vorgeschlagenen Verfahren hergestellt werden. Dabei wird die Verbindung der Formel Ia in einer Nährlösung, die eine Kohlenstoffquelle und eine Stickstoffquelle, sowie übliche anorganische Salze enthält, von Penicillium spec., bevorzugt DSM 4209 oder DSM 4210, produziert. Anstelle der Stämme DSM 4209 oder DSM 4210 können natürlich auch deren Mutanten und Varianten eingesetzt werden, soweit sie diese Verbindungen synthetisieren.

Die Bildung der Verbindung der Formel Ia verläuft besonders gut in einer Nährlösung, die etwa 0,2 bis 5 %, bevorzugt 1 bis 4 %, Malzextrakt, 0,02 bis 0,5 %, bevorzugt 0,1 bis 0,4 % Hefeextrakt, 0,1 bis 5 %, bevorzugt 0,5 bis 2 % Glucose und 0,005 bis 0,2 %, bevorzugt 0,01 bis 0,1 % Ammoniumsalz enthält, jeweils bezogen auf das Gewicht der gesamten Nährlösung. Die Kultivierung erfolgt aerob, also beispielsweise submers unter Schütteln oder Rühren in Schüttelkolben oder Fermentern, gegebenenfalls unter Einführen von Luft oder Sauerstoff. Sie kann in einem Temperaturbereich von etwa 18 bis 35 °C, vorzugsweise bei etwa 25 bis 30 °C, insbesondere bei 27 bis 28 °C durchgeführt werden. Der pH-Bereich sollte zwischen 2 und 8 liegen, vorteilhaft zwischen 3 und 7. Man kultiviert den Mikroorganismus unter diesen Bedingungen im allgemeinen über einen Zeitraum von 60 bis 170 Stunden, bevorzugt 100 bis 150 Stunden.

Zur Isolierung der Verbindung der Formel Ia werden Kulturbrühe und Mycel zuerst mit organischen Lösungsmitteln, wie z.B. Chloroform, Essigester usw. extrahiert, um die unpolaren Verunreinigungen zu entfernen. Anschließend wird mit einem polaren Lösungsmittel, beispielsweise niederen Alkanolen oder Gemischen aus Chloroform und/oder Essigester mit einem niederen Alkanol, extrahiert.

Die Reinisolierung erfolgt vorzugsweise an geeigneten Medien, wie z.B. Kieselgel, Aluminiumoxid oder Ionenaustauschern, durch anschließende Elution mit organischen, polaren Lösungsmitteln oder Lösungsmittelgemischen.

Im folgenden werden die Verfahren a) bis h), die es ermöglichen die unterschiedlich substituierten Decarestrictine der Formel I herzustellen, näher beschrieben.

Bei der Verfahrensvariante a) geht man am besten so vor, daß man die Verbindung der Formel Ia in äquimolaren Mengen oder in bis zu einem 50-fachen Überschuß, gegebenenfalls in einem inerten, aprotischen Lösungsmittel, wie Chloroform, Methylenchlorid, Tetrahydrofuran (THF), Essigester oder Dioxan, mit einer Verbindung der Formel IV bis zur Beendigung der Reaktion umsetzt, gegebenenfalls in Gegenwart einer Base, vorzugsweise Pyridin oder Triethylamin. Setzt man eine Verbindung der Formel IV mit Q = OH ein, so empfiehlt sich die Zugabe von Dicyclohexylcarbodiimid, gegebenenfalls in Anwesenheit eines Katalysators wie N,N-Dimethylaminopyridin (DMAP). Die Reaktionstemperaturen liegen dabei zwischen -70°C und +100°C, vorzugsweise bei Verwendung eines Lösungsmittels zwischen dem Festpunkt und dem Siedepunkt des Lösungsmittels, insbesondere zwischen -70°C und +40°C. Die Reaktionszeiten betragen 1 bis 180 Stunden, bevorzugt 1 bis 48 Stunden, besonders bevorzugt 1 bis 8 Stunden. Die Beendigung der Reaktion kann beispielsweise mittels Dünnschichtchromatographie bestimmt werden (DC-Kontrolle).

Die Ausgangsverbindungen für die Verfahrensvariante a), die Verbindungen der Formel IV sind, sofern nicht käuflich, auf einfache Weise nach literaturbekannten Verfahren herstellbar. Beispielsweise erhält man die Säurechloride durch Umsetzung der entsprechenden Carbonsäure mit Thionylchlorid, $PCl_3$ oder $PCl_5$.

Bei der Verfahrensvariante b) geht man am besten so vor, daß man die Verbindung der Formel Ia in einem Lösungsmittel löst, beispielsweise Essigester, Dimethoxyethan, Tetrahydrofuran, tert. Butylmethylether oder Methylethylketon, und dann den Benzoyl- oder Acyldonor, z.B. einen Carbonsäureester, bevorzugt einen Vinylester, wie beispielsweise Vinylacetat, Benzoesäurevinylester, Chloressigsäurevinylester, Methoxyessigsäurevinylester, Phenoxyessigsäurevinylester, Phenylessigsäurevinylester, Aminosäurevinylester, z.B. Z-Glycinvinylester oder Isopropenylacetat hinzufügt oder anstelle eines Carbonsäureesters die Carbonsäureanhydride, wie z.B. Pivalinsäureanhydrid, Essigsäureanhydrid oder Benzoesäureanhydrid. Man kann aber auch den Benzoyl- oder Acyldonor gleichzeitig als Lösungsmittel benutzen. Geeignete Enzyme sind Lipasen oder Esterasen, die in freier oder immobilisierte Form zugegeben werden, wobei bei Temperaturen zwischen 0 und 80°C, vorzugsweise zwischen 10 und 50°C, inkubiert wird.

Zur Immobilisierung der Enzyme kommen alle gängigen, in der Literatur beschriebenen Verfahren in Frage [W. Hartmeier, Trends in Biotechnology 3, 149 (1985); A. Rosevaer, J. Chem. Tech. Biotechnol. 34B, 127 (1984)]. Immobilisierte und freie Enzyme können auch im Säulenverfahren eingesetzt werden.

Die Reaktionszeiten sind von Struktur und Löslichkeit der Verbindungen der Formel I, von der Temperatur und von den Konzentrationsverhältnissen, insbesondere der Enzymmenge abhängig. Sie können zwischen 0,5 Stunden und 3 Tagen liegen, jedoch in aller Regel zwischen 5 und 24 Stunden.

Die Aufarbeitung der Reaktionsprodukte erfolgt meist durch Abtrennen, z.B. Abfiltrieren, der freien oder immobilisierten Enzyme, Abdestillieren der Lösungsmittel und der Acyldonatoren im Vakuum und Reinigung der Produkte, sofern nötig, durch Kristallisation, Chromatographie oder Extraktion.

Erfindungsgemäß können Lipasen und Esterasen in dem Verfahren eingesetzt werden, die vorzugsweise aus Mikroorganismen bzw. Pankreas oder Leber von Tieren gewonnen werden. Insbesondere bevorzugt werden Lipasen und Esterasen aus Pseudomonas, Candida, Mucor, Rhizopus, Penicillium oder Aspergillus, sowie aus Schweineleber oder Schweinepankreas verwendet. Die Enzyme sind käuflich bzw. können nach bekannten Methoden aus dem entsprechenden Mikroorganismus bzw. aus der Leber oder dem Pankreas extrahiert werden.

Bei der Verfahrensvarianten c) geht man am besten so vor, daß man die Verbindung der Formel Ia in äquimolaren Mengen oder in bis zu einem 50-fachen Überschuß, gegebenenfalls in einem inerten Lösungsmittel wie Chloroform, Tetrachlorkohlenstoff, Methylenchlorid oder Hexan mit einem Oxidationsmittel wie Braunstein, Pyridiniumchlorochromat oder Pyridiniumdichromat bis zur Beendigung der Reaktion umsetzt.

Die Reaktionstemperaturen liegen dabei zwischen -70°C und +100°C, vorzugsweise bei Verwendung eines Lösungsmittels zwischen dem Festpunkt und dem Siedepunkt des Lösungsmittels, insbesondere zwischen -70°C und +40°C. Die Reaktionszeiten betragen 1 bis 180 Stunden, bevorzugt 1 bis 48 Stunden, besonders bevorzugt 1 bis 8 Stunden. Die Beendigung der Reaktion kann beispielsweise mittels Dünnschichtchromatographie (DC) bestimmt werden.

Die Oxidationsmittel für die Verfahrensvariante c) sind käuflich.

Bei der Verfahrensvarianten d) geht man am besten so vor, daß man die Verbindung der Formel Ia in äquimolaren Mengen oder in bis zu einem 50-fachen Überschuß, gegebenenfalls in einem inerten Lösungsmittel, vorzugsweise einem halogenierten Kohlenwasserstoff, wie z.B. Dichlormethan, mit $H_2O_2$, organischen

Persäuren, wie z.B. m-Chlorperbenzoesäure, oder Mischungen aus $H_2O_2$ mit organischen Säuren umsetzt.

Die Reaktionstemperatur liegt im allgemeinen zwischen -30 bis +30 °C, vorzugsweise zwischen -10 bis +5 °C. Die Reaktionszeiten betragen 30 min bis 24 Stunden, bevorzugt 1 bis 10 Stunden. Die Beendigung der Reaktion kann beispielsweise durch DC bestimmt werden.

Bei der Verfahrensvarianten e) geht man am besten so vor, daß man die Verbindung der Formel Ia in einem inerten Lösungsmittel, z.B. niederen Alkoholen, wie Methanol, Ethanol oder aliphatischen oder cyclischen Ethern, wie Tetrahydrofuran (THF), oder Mischungen aus inerten Lösungsmitteln löst und unter Verwendung eines gängigen Hydrierkatalysators, z.B. Pd- oder Pt-Katalysatoren auf Aktivkohle, nach literaturbekannten Verfahren hydriert.

Bei der Verfahrensvariante f) geht man am besten so vor, daß man eine Verbindung der Formel Ia in äquimolaren Mengen oder in bis zu einem 50-fachen Überschuß mit einem Aldehyd oder Keton, wie z.B. Acetaldehyd, Propionaldehyd, Butyraldehyd, Aceton oder Benzaldehyd, gegebenenfalls in einem inerten Lösungsmittel, wie Chloroform, Methylenchlorid, Nitromethan oder Acetonitril in Gegenwart von Zinkchlorid umsetzt.

Die Reaktionstemperaturen liegen zwischen -70 °C und +100 °C, vorzugsweise bei Verwendung der Ketone oder Aldehyde als Lösungsmittel zwischen dem Festpunkt und dem Siedepunkt des Lösungsmittels, insbesondere zwischen 0 °C und +40 °C. Die Reaktionszeiten betragen 1 bis 180 Stunden, bevorzugt 1 bis 72 Stunden, insbesondere 1 bis 48 Stunden. Die Beendigung der Reaktion kann beispielsweise mittels Dünnschichtchromatographie bestimmt werden.

Die Verbindungen

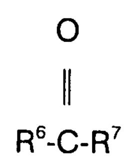

oder Benzaldehyd sind, sofern nicht käuflich, auf einfache Weise nach literaturbekannten Verfahren herstellbar.

Bei der Verfahrensvariante g) geht man am besten so vor, daß man die Verbindung der Formel VIII oder IV in äquimolaren Mengen oder in bis zu einem 50-fachen Überschuß, gegebenenfalls in einem inerten Lösungsmittel, wie Chloroform, Methylenchlorid, Nitromethan, Acetonitril oder Toluol, mit einer Verbindung der Formel X bis zur Beendigung der Reaktion in Anwesenheit eines Katalysators wie Tetraethylammoniumbromid/Molsieb 4Å, $Hg(CN)_2$, $Ag_2CO_3$, $AgClO_4$ oder Silbertrifluormethansulfonat umsetzt, gegebenenfalls in Gegenwart einer Base, wie Collidin, Lutidin oder Tetramethylharnstoff.

Die Reaktionstemperaturen liegen dabei zwischen -70 °C und +100 °C, vorzugsweise bei Verwendung eines Lösungsmittels zwischen dem Festpunkt und dem Siedepunkt des Lösungsmittels, insbesondere zwischen -70 °C und +40 °C. Die Reaktionszeiten betragen 1 bis 180 Stunden, bevorzugt 1 bis 48 Stunden besonders bevorzugt 1 bis 8 Stunden. Die Beendigung der Reaktion kann beispielsweise mittels Dünnschichtchromatographie bestimmt werden.

Die Ausgangsverbindungen für die Verfahrensvariante g), die Verbindungen der Formel X sind, sofern nicht käuflich, auf einfache Weise nach literaturbekannten Verfahren herstellbar. Bevorzugt werden in 2-Position halogensubstituierte Mono- oder Disaccharide eingesetzt.

Bei der Verfahrensvarianten h) geht man am besten so vor, daß man die Verbindung der Formel Ia in äquimolaren Mengen oder in bis zu einem 50-fachen Überschuß, gegebenenfalls in einem inerten Lösungsmittel wie Chloroform, Methylenchlorid, Tetrahydrofuran (THF), Essigester oder Dioxan, mit einer Verbindung der Formel XI bis zur Beendigung der Reaktion umsetzt.

Die Reaktionstemperaturen liegen dabei zwischen -70 °C und +100 °C, vorzugsweise bei Verwendung eines Lösungsmittels zwischen dem Festpunkt und dem Siedepunkt des Lösungsmittels, insbesondere zwischen -70 °C und +40 °C. Die Reaktionszeiten betragen 1 bis 180 Stunden, bevorzugt 1 bis 48 Stunden, besonders bevorzugt 1 bis 8 Stunden. Die Beendigung der Reaktion kann beispielsweise mittels Dünnschichtchromatographie bestimmt werden.

Die Ausgangsverbindungen für die Verfahrensvariante h), die Verbindungen der Formel XI sind, sofern nicht käuflich, auf einfache Weise nach literaturbekannten Verfahren herstellbar.

Als Ausgangssubstanz für die Umsetzungen gemäß der Verfahrensvarianten a) bis h) kann nicht nur die Verbindung der Formel Ia verwendet werden, sondern auch eine bereits nach einem oder mehreren der Verfahren a) bis h) derivatisierte Verbindung. Auf diese Weise lassen sich nacheinander die verschiedensten Substituenten $R^1$, $R^2$, $R^3$, $R^4$ und $R^5$ in das Grundgerüst der Formel I einführen.

Die Reinigung, Isolierung und Aufarbeitung der nach den Verfahrensvarianten a) bis h) erhaltenen Substanzen erfolgt nach den üblichen Methoden; beispielsweise können die Reaktionsprodukte durch Chromatographie an polaren Trägermaterialien wie Kieselgel oder ®Sephadex LH 20 mit Lösungsmitteln wie niederen Alkanolen wie Methanol oder Chloroform, Dichlormethan oder Essigester oder Methanol/Chloroform-Mischungen oder Chloroform/Heptan/Methanol-Mischungen aber auch durch extraktive Methoden wie flüssig/flüssig-Extraktion oder fest/flüssig-Extraktion oder durch Kristallisation gereinigt werden.

Die erfindungsgemäßen Verbindungen der Formel I zeigen antibakterielle und antivirale Wirkung. Die antivirale Aktivität wurde in Zellkulturen, die mit Testviren infiziert wurden, getestet. Die erfindungsgemäßen Verbindungen sind aufgrund ihrer pharmakologischen Eigenschaften für die Behandlung von bakteriellen Erkrankungen und Viruserkrankungen geeignet.

Die Inhibierung der Cholesterin-Biosynthese durch die erfindungsgemäßen Verbindungen, sowie deren physiologisch verträgliche Salze wurde in-vitro-Tests überprüft. Dabei zeigte sich, daß die erfindungsgemäßen Derivate eine hervorragende Wirkung als Inhibitoren der Cholesterin-Biosynthese zeigen. Sie können dementsprechend als Lipidsenker eingesetzt werden. Die erfindungsgemäßen Verbindungen sind aufgrund ihrer pharmakologischen Eigenschaften für die Behandlung von Störungen des Stoffwechsels von Cholesterin und cholesterinähnlichen Stoffen geeignet.

Die Erfindung betrifft daher ferner die Anwendung der erfindungsgemäßen Verbindungen der Formel I, sowie deren physiologisch verträglichen Salze bei der Behandlung und Prophylaxe von Störungen des Stoffwechsels von Cholesterin und cholesterinähnlichen Stoffen.

Die Verbindungen können entweder allein oder mit physiologisch verträglichen Hilfs- oder Trägerstoffen vermischt als Arzneimittel angewandt werden. Sie können zu diesem Zweck oral in Dosen von 0,01 - 5,0 mg/kg/Tag, vorzugsweise 0,01 - 1,0 mg/kg/Tag oder parenteral subkutan in Dosen von 0,001 - 2,5 mg/kg/Tag, vorzugsweise 0,001 - 1,0 mg/kg/Tag, insbesondere 0,005 - 0,2 mg/kg/Tag, appliziert werden. Die Dosierung kann in schweren Fällen auch erhöht werden. In vielen Fällen genügen jedoch auch geringere Dosen. Diese Angaben beziehen sich auf einen Erwachsenen von etwa 75 kg Gewicht.

Die Erfindung umfaßt weiterhin die Verwendung der erfindungsgemäßen Verbindungen bei der Herstellung von Arzneimitteln, die zur Behandlung und Prophylaxe der vorstehend genannten Krankheiten eingesetzt werden.

Ein weiterer Gegenstand der Erfindung sind Arzneimittel, die ein oder mehrere erfindungsgemäße Verbindungen der Formel I und/oder deren physiologisch verträgliche Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche oder vorzugsweise in Kombination mit geeigneten pharmazeutischen Zusatz, Hilfs- oder Trägerstoffen in Form von Tabletten, Dragees, Kapseln, Suppositorien, Emulsionen, Suspensionen oder Lösungen eingesetzt, wobei der Wirkstoffgehalt bis etwa 95 %, vorteilhafterweise zwischen 10 und 75 % beträgt.

Geeignete Hilfs- bzw. Trägerstoffe für die gewünschte Arzneimittelformulierung sind beispielsweise neben Lösemitteln, Gelbildnern, Suppositoriengrundlagen, Tabletten-Hilfsstoffe und anderen Wirkstoffträgern auch Antioxidantien, Dispergiermittel, Emulgatoren, Entschäumer, Geschmackskorrigentien, Konservierungsmittel, Lösungsvermittler oder Farbstoffe.

Die Wirkstoffe können oral, parenteral, subkutan oder rectal appliziert werden.

Die aktiven Verbindungen werden mit den dafür geeigneten Zusatzstoffen wie Trägerstoffen, Stabilisatoren oder inerten Verdünnungsmitteln vermischt und durch die üblichen Methoden in geeignete Darreichungsformen gebracht, wie Tabletten, Dragees, Steckkapseln, wäßrige, alkoholische oder ölige Suspensionen oder wäßrige oder ölige Lösungen. Als inerte Trägerstoffe können z.B. Gummi arabicum, Magnesia, Magnesiumcarbonat, Kaliumphosphat, Milchzucker, Glukose oder Stärke, insbesondere Maisstärke, verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht, wie Sonnenblumenöl oder Lebertran.

Zur subkutanen oder intravenösen Applikation werden die aktiven Verbindungen gewünschtenfalls mit den dafür geeigneten Substanzen wie Lösungsvermittler, Emulgatoren oder weiteren Hilfsstoffen in Lösung, Suspension oder Emulsion gebracht. Als Lösungsmittel kommen z.B. in Frage physiologische Kochsalzlösung oder Alkohole, z.B. Ethanol, Propanol, Glycerin, daneben auch Zuckerlösungen wie Glucose- oder Mannitlösungen, oder auch eine Mischung aus den verschiedenen genannten Lösungsmitteln.

Nachfolgend ist die Erfindung an Hand von Beispielen näher erläutert.

Alle nach den folgenden Beispielen erhaltenen Verbindungen wurden mittels C,H-Analyse und/oder $R_1$-Wert charakterisiert. Die Verbindung der Formel Ia wurde gemäß der EP-A-89 104 160 hergestellt.

Herstellung der Verbindung der Formel Ia

a) Herstellung der Sporensuspension des Produzentenstammes:

100 ml Nährlösung (2 g Hefeextrakt, 20 g Malzextrakt, 10 g Glucose, 0,5 g $(NH_4)_2PO_4$, 1 l Leitungswasser, pH-Wert vor dem Sterilisieren 7,3) in einem 500 ml Erlenmeyerkolben werden mit dem Stamm DSM 4209 oder DSM 4210 beimpft und 72 Stunden bei 25 ° C und 120 UpM auf der rotierenden Schüttelmaschine inkubiert. Anschließend werden 20 ml Kulturflüssigkeit in einem 500 ml Erlenmeyerkolben mit dem Nährboden der obengenannten Zusammensetzung, dem 20 g Agar/l zur Verfestigung zugegeben wurde, gleichmäßig verteilt und dekantiert. Die Kulturen werden 10 bis 14 Tage bei 25 ° C inkubiert. Die nach dieser Zeit entstandenen Sporen eines Kolbens werden mit 500 ml entionisiertem Wasser, das einen Tropfen eines handelsüblichen nichtionischen Tensids (Triton X 100, Fa. Serva) enthält, abgeschwemmt, sofort weiterverwendet oder bei -22 ° C aufbewahrt.

b) Herstellung einer Kultur bzw. Vorkultur des Produzentenstammes im Erlenmeyerkolben:

Ein 500 ml Erlenmeyerkolben mit 100 ml der unter a) beschriebenen Nährlösung wird mit einer auf einem Schrägröhrchen gezogenen Kultur oder mit 0,2 ml Sporensuspension angeimpft und auf einer Schüttelmaschine bei 120 UpM und 25 ° C inkubiert. Die maximale Produktion ist nach ca. 120 Stunden erreicht. Zum Animpfen von 10 und 100 l Fermentern genügt eine 48 Stunden alte Submerskultur (5 %) aus der gleichen Nährlösung.

c) Herstellung der Verbindung der Formel Ia

Ein 10 l Fermenter wird unter folgenden Bedingungen betrieben:

| | |
|---|---|
| Nährmedium | 20 g/l Malzextrakt<br>2 g/l Hefeextrakt<br>10 g/l Glucose<br>0,5 g/l $(NH_4)_2PO_4$<br>pH 3,5 |
| Inkubationszeit:<br>Inkubationstemperatur:<br>Rührergeschwindigkeit:<br>Belüftung: | 150 Stunden<br>25 ° C<br>250 UpM<br>4 l Luft/min. |

Durch wiederholte Zugabe weniger Tropfen ethanolischer Polyollösung kann die Schaumentwicklung unterdrückt werden. Das Produktionsmaximum wird nach ca. 150 Stunden erreicht. Die Ausbeuten liegen bei ca. 100 mg/l.

d) Isolierung der Verbindung der Formel Ia

Nach Fermentation von DSM 4209 bzw. DSM 4210 wird die Kulturbrühe unter Zusatz von 2 % ®Celite als Filterhilfsmittel filtriert. Die Verbindung der Formel Ia wird aus dem Kulturüberstand isoliert. Dazu wird der Kulturüberstand lyophilisiert, zuerst über Mitteldruck-Flüssigchromatographie in Chloroform/Methanol (40:1) chromatographiert und anschließend mit Normaldruck-Säulenchromatographie in Chloroform/Methanol (20:1) gereinigt. Nach Säulenchromatographie mit ®Sephadex erhält man die Verbindung der Formel Ia.

Beispiel 1

Herstellung von 10-Methyl-1-oxa-4,5,8-tris-(n-butylcarbonyl-oxy)-6-cyclodecen-2-on

100 mg der Ausgangsverbindung Ia (0,46 mmol) werden in einer Lösung aus 0,7 ml Acetanhydrid und 2 ml abs. Pyridin gelöst und das Reaktionsgemisch 24 h bei 25 ° C gerührt.

Anschließend wird das Reaktionsgemisch im Vakuum eingeengt und der ölige Rückstand mit 20 ml Dichlormethan aufgenommen. Die erhaltene organische Phase wird dreimal mit jeweils 5 ml Wasser extrahiert, über $Na_2SO_4$ getrocknet und im Vakuum eingeengt. Das so erhaltene Rohprodukt wird an Kieselgel gereinigt (Laufmittel: $CHCl_3$:Methanol = 40:1).

Ausbeute 131 mg; 0,38 mmol (83 %), farbloses Öl.

Beispiel 2

Herstellung von 8-Acetyloxy-4,5-dihydroxy-10-methyl-1-oxa-6-cyclodecen-2-on

100 mg (0,46 mmol) der Ausgangsverbindung Ia werden in 10 ml eines 1:2 Lösungsmittelgemisches aus Dimethoxyethan/Vinylacetat aufgenommen und mit etwa 20 mg Lipase P/FP (Pseudomonas fluoreszens; Fa. Amano; Pharmaceutical Co., Ltd., Nagoya, Japan) versetzt und das Reaktionsgemisch wird bei 25° C für 72 Stunden gerührt.
Der Verlauf der Reaktion wird dünnschichtchromatographisch verfolgt (Kieselgel-DC-platten, Laufmittel $CHCl_3$:Hexan:Methanol = 4:4:1).
Zur Aufarbeitung wird das Enzym über Membranfilter abfiltriert, eingeengt und das Rohprodukt chromatographiert (Kieselgelsäule, Laufmittel:$CHCl_3$:Methanol = 19:1). Ausbeute: 85,5 mg; 0,33 mmol (72 %), farbloses Öl.

Beispiel 3

Herstellung von 4-Hydroxy-10-methyl-1-oxa-6-cyclodecan-2,5,8-trion

74 mg der Ausgangsverbindung Ia (0,34 mmol) werden in 10 ml Dichlormethan gelöst und mit 236,6 mg Braunstein (8 Äquivalente; 2,72 mmol) versetzt und das Gemisch 12 Stunden bei 25° C gerührt. (DC-Kontrolle).
Zur Aufarbeitung wird über Filterhilfsmittel filtriert und die Reaktionslösung im Vakuum eingeengt.
Das Rohprodukt wird chromatographiert (Laufmittel: Ethylacetat:Methanol = 19:1). Ausbeute: 33,2 mg; 0,16 mmol (46 %), farbloses Öl.

Beispiel 4

Herstellung von 10-Methyl-1-oxa-4,5,8-trihydroxy-2-carbonyl-6-cyclodecenoxid

75 mg (0,35 mmol) der Ausgangsverbindung Ia werden in 12 ml absoluten $CH_2Cl_2$ gelöst und bei -5° C mit 51 mg (0,37 mmol) m-Chlorperbenzoesäure, gelöst in 5 ml $CH_2Cl_2$, innerhalb von 30 Minuten versetzt. Das Reaktionsgemisch wird 8 h bei 0° C gerührt. Zur Aufarbeitung wird die organische Phase dreimal mit 5 ml $H_2O$ gewaschen, über $Na_2SO_4$ getrocknet und im Vakuum eingeengt.
Das Rohprodukt wird chromatographisch gereinigt (Laufmittel: Chloroform:Methanol = 9:1).
Ausbeute: 74,8 mg; 0,32 mmol (92 %), farbloses Öl.

Beispiel 5

Herstellung von 8-Acetyloxy-4,5-dihydroxy-10-methyl-1-oxa-cyclodecan-2-on

150 mg (0,69 mmol) der Verbindung nach Beispiel 19 werden in 15 ml Methanol gelöst und mit 15 mg 5 % Palladium auf Kohle (Fa. Hedrich) versetzt und unter Wasserstoffatmosphäre bis zur Beendigung der Wasserstoff-Aufnahme bei 25° C gerührt. Zur Aufarbeitung wird das Reaktionsgemisch über Filterhilfsmittel filtriert und anschließend im Vakuum eingeengt. Das Rohprodukt wird chromatographiert (Laufmittel: Hexan:$CHCl_3$:Methanol = 4:4:1; Kieselgel).
Ausbeute: 128 mg; 0,59 mmol (85 %), farbloses Öl.

Beispiel 6

Herstellung von 10-Methyl-4,5,8-trihydroxy-4,5-O-isopropyliden-1-oxacyclodecan-2-on

104 mg der Verbindung aus Beispiel 15 (0,46 mmol) werden in 20 ml Aceton suspendiert und die Lösung mit 250 mg wasserfreiem $ZnCl_2$ versetzt und das Reaktionsgemisch bei 25° C, 2 Tage gerührt. Der Verlauf der Reaktion wird dünnschichtchromatographisch verfolgt (Kieselgelplatten, Laufmittel: Chloroform/Hexan/Methanol = 8:8:1).
Nach Beendigung der Reaktion (etwa 2 Stunden) wird das Lösungsmittel im Vakuum entfernt und der verbleibende Rückstand über Kieselgel chromatographiert (Laufmittel:Methylenchlorid:Heptan:Methanol = 8:8:1).
Ausbeute: 79 mg; 0,31 mmol (67 %), farbloses Öl.

Beispiel 7

Herstellung von 4,5-Dihydroxy-O-isopropyliden-10-methyl-1-oxa-8(2,3,4,6-tetra-O-benzyl-α-D-glucopyranosyl-oxy)-2-cyclodecanon

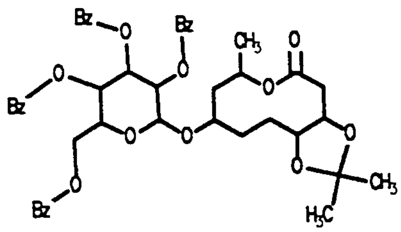

(Bz entspricht $-CH_2$-phenyl) 40 mg (0,155 mmol) 10-Methyl-1-oxa-4,5,8-trihydroxy-4,5-di-O-isopropyliden-2-cyclodecanon (hergestellt gemäß Beispiel 17) werden in 4 ml absolutem Acetonitril gelöst und nach Zugabe von 102 µl 2,4,6-Collidin (0,78 mmol, 5 Äquivalente) sowie 1 g fein gepulvertem Molekularsieb (3 Å) (Aldrich, St einheim, BRD) 30 Minuten bei Raumtemperatur gerührt ($N_2$-Schutzgasatmosphäre). Anschließend wird mit 112 mg (0,201 mmol, 1,3 Äquivalente) 2,3,4,6-Tetra-O-benzyl-α-D-glucopyran osyl-chlorid (Herstellung s. V.D. Grob et. al.; Carbohyd. Res., 10 (1969) 595-597) und 52 mg (0,202 mmol, 1,3 Äquivalente) Silber-trifluormethan-sulfonat versetzt und das Reaktionsgemisch 72 Stunden bei Raumtemperatur gerührt. Der Verlauf der Reaktion wird dünnschichtchromatographisch verfolgt (Laufmittel: $CHCl_3$:Hexan:Methanol = 20:20:1).

Zur Aufarbeitung wird über Filterhilfsmittel filtriert und das Filtrat im Vakuum eingeengt. Der Rückstand wird an Kieselgel chromatographiert (Laufmittel: $CH_2Cl_2$:Ethylacetat = 1:1).
Ausbeute: 32,7 mg; 0,042 mmol (27 %), farbloses Öl.

Beispiel 8

Herstellung von 10-Methyl-4,5,8-trimethoxy-1-oxa-6-cyclodecen-1-on

80 mg (0,37 mmol) der Ausgangsverbindung Ia werden in 10 ml absolutem Dimethylformamid gelöst und bei 25 °C mit 0,22 ml Jodmethan (14,8 mmol; 404 mg) und 430 mg (1,85 mmol) Silber(I)oxid versetzt und das Reaktionsgemisch 24 Stunden bei 25 °C unter Lichtausschluß gerührt. Der Verlauf der Reaktion wird dünnschichtchromatographisch verfolgt (Laufmittel: $CH_2Cl_2$:Ethylacetat = 4:1). Zur Aufarbeitung wird über Filterhilfsmittel (Celite) filtriert und das Filtrat im Vakuum eingeengt.
Anschließend wird das Rohprodukt über Kieselgel chromatographiert. (Laufmittel: $CH_2Cl_2$:Ethylacetat = 6:1).
Ausbeute: 46,1 mg; 18 mmol, (48 %) farbloses Öl.

Die nachfolgende Tabelle 1 gibt eine Übersicht über die hergestellten Verbindungen und über solche, die analog zu den Beispielen 1 bis 8 hergestellt wurden. Tabelle 2 zeigt die C,H-Analyse der hergestellten Verbindungen und deren $R_f$-Wert bei der Chromatographie in Kieselgel.

Tabelle 1

| Beispiel Nr. | Verbindung | hergestellt aus | Reagenz Bedingungen | Bedingung | Verfahren | Ausführung gemäß Bsp. | Ausbeute |
|---|---|---|---|---|---|---|---|
| 1 | 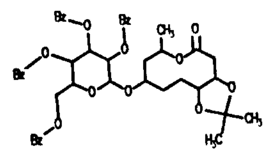 | 17 | 2-Chlor-tetrabenzyl-α-D-tetrabenzylglucose; Silbertriflat, Collidin, Molsieb (3 Å), $CH_3CN$ | 6 h, RT | g) | 7 | 27 % |
| 2 | 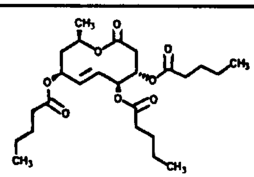 | Ia | Valeriansäureanhydrid, Pyridin | 24 h, RT | a) | 1 | 87 % |
| 3 | 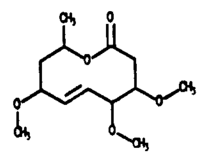 | Ia | Me I, $Ag_2O$, DMF, Lichtausschluß | 24 h, RT | h) | 8 | 48 % |
| 4 | 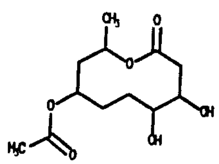 | 19 | Pd (5 % auf Kohle), THF, $H_2$ | 4 h, RT | e) | 5 | 62 % |

RT = 25°C

h = Stunden

| Beispiel Nr. | Verbindung | hergestellt aus | Reagenz Bedingungen | Bedingung | Verfahren | Ausführung gemäß Bsp. | Ausbeute |
|---|---|---|---|---|---|---|---|
| 5 | 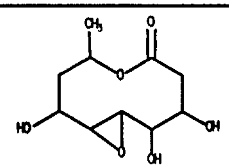 | Ia | m-CPBA, $CH_2Cl_2$ | | d) | 4 | 92 % |
| 6 | 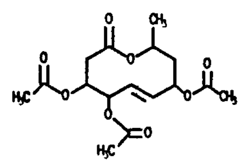 | Ia | Acetanhydrid, Pyridin | 24 h, RT | a) | 1 | 83% |
| 7 | 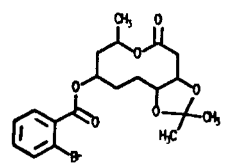 | 17 | o-Brombenzoesäure, DCC, DMAP, $CH_2Cl_2$ | 18 h, RT | a) | 1 | 72 % |
| 8 | 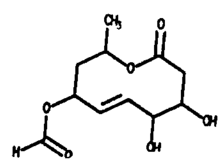 | Ia | Ameisensäure | 24 h, RT | a) | 1 | 80 % |

| Beispiel Nr. | Verbindung | hergestellt aus | Reagenz Bedingungen | Bedingung | Verfahren | Ausführung gemäß Bsp. | Ausbeute |
|---|---|---|---|---|---|---|---|
| 9 | 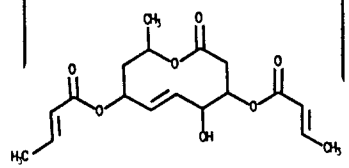 | Ia | Crotonsäureanhydrid Pyridin, DMAP, $CH_2Cl_2$ | 24 h, RT | a) | 1 | 35 % |
| 10 | 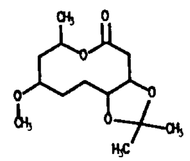 | 1.7 | $CH_3I$, DMF, $Ag_2O$ | 18 h, RT | h) | 8 | 48 % |
| 11 | 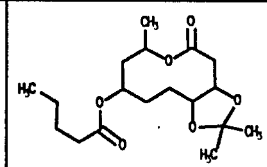 | 17 | Valeriansäureanhydrid, Pyridin | 18 h, RT | a) | 1 | 83 % |
| 12 | 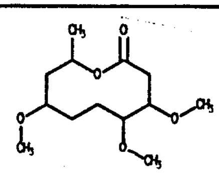 | 3 | Pd (5 % auf Kohle), THF, $H_2$ | 6 h, RT | e) | 5 | 68 % |

h = Stunde

RT = 25°C

| Bei-spiel Nr. | Verbindung | herge-stellt aus | Reagenz Bedingungen | Bedingung | Verfahren | Ausführung gemäß Bsp. | Ausbeute |
|---|---|---|---|---|---|---|---|
| 13 | 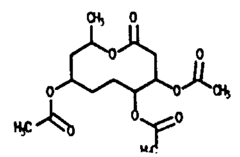 | 6 | Pd (5 % auf Kohle), THF, $H_2$ | 6 h, RT | e) | 5 | 61 % |
| 14 | 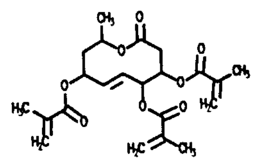 | Ia | Methacrylsäureanhydrid, Pyridin | 12 h, RT | a) | 1 | 76 % |
| 15 | 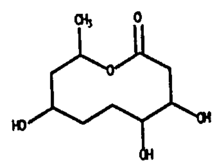 | Ia | Pd (5 % auf Kohle), MeOH | | e) | 5 | 85 % |
| 16 | 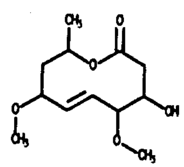 | Ia | Me I, $Ag_2O$, DMF, Lichtausschluß | 12 h, RT | h) | 8 | 30 % |

| Bei-spiel Nr. | Verbindung | herge-stellt aus | Reagenz Bedingungen | Bedingung | Verfahren | Ausführung gemäß Bsp. | Ausbeute |
|---|---|---|---|---|---|---|---|
| 17 | 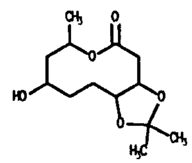 | 15 | Aceton, $ZnCl_2$ | 2 h, RT | f) | 6 | 67 % |
| 18 | 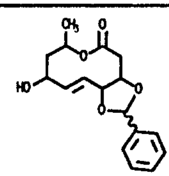 | Ia | Benzaldehyd, $ZnCl_2$, $CH_2Cl_2$ | 4 h, RT | f) | 6 | 41 % |
| 19 | 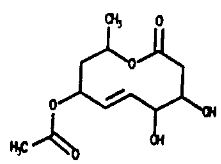 | Ia | Lipase P/FP, Vinylacetat, DME | 72 h, RT | b) | 2 | 72 % |
| 20 | 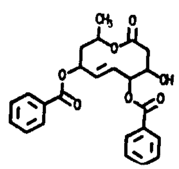 | Ia | Benzoesäureanhydrid, DMAP, Pyridin | 12 h, RT | a) | 1 | 54 % |

| Bei-spiel Nr. | Verbindung | herge-stellt aus | Reagenz Bedingungen | Bedingung | Verfahren | Ausführung gemäß Bsp. | Ausbeute |
|---|---|---|---|---|---|---|---|
| 21 | 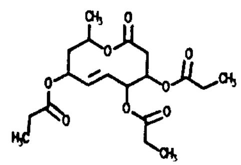 | Ia | Propionsäureanhydrid, Pyridin | 18 h, RT | a) | 1 | 87 % |
| 22 | 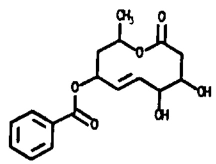 | Ia | Benzoesäureanhydrid, Pyridin, DMAP | 4 h, RT | a) | 1 | 40 % |
| 23 | 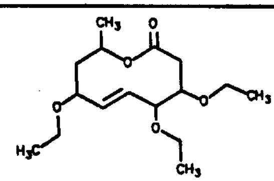 | Ia | Jodethan, $Ag_2O$, $CH_3CN$ | 18 h, RT | h) | 8 | 74 % |
| 24 | 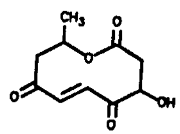 | Ia | $MnO_2$, $CH_2Cl_2$ | 18 h, RT | c) | 3 | 46 % |

Tabelle 2

| Verb. Nr. | C, H ber. | | C, H gef. | | $R_f$ (Laufmittel) (Kieselgel) |
|---|---|---|---|---|---|
| 1 | C 72,3 | H 7,2 | C 77,2 | H 7,0 | 0,41 ($CHCl_3$:Hexan:Methanol = 20:20:1) |
| 2 | C 64,1 | H 8,6 | C 64,2 | H 8,5 | 0,47 ($CHCl_3$) |
| 3 | C 60,5 | H 8,6 | C 60,4 | H 8,8 | 0,54 ($CH_2Cl_2$: Ethylacetat = 4:1) |
| 4 | C 55,4 | H 7,7 | C 55,4 | H 7,6 | 0,53 ($CHCl_3$:Hexan:Methanol = 4:4:1) |
| 5 | C 51,7 | H 6,9 | C 51,6 | H 6,8 | 0,38 ($CH_2Cl_2$:Methanol = 9:1) |
| 6 | C 56,1 | H 6,5 | C 56,2 | H 6,7 | 0,4 ($CHCl_3$) |
| 7 | C 54,4 | H 5,7 | C 54,2 | H 5,6 | 0,48 ($CH_2Cl_2$:Ethylacetat = 19:1) |
| 8 | C 54,1 | H 6,5 | C 54,0 | H 6,5 | 0,76 ($CH_2Cl_2$:Methanol = 9:1) |
| 9 | C 61,4 | H 6,9 | C 61,2 | H 6,8 | 0,62 ($CH_2Cl_2$:Hexan:Methanol = 8:8:1) |
| 10 | C 61,7 | H 8,9 | C 61,7 | H 9,0 | 0,53 ($CH_2Cl_2$:Ethylacetat = 8:1) |
| 11 | C 63,1 | H 8,8 | C 63,2 | H 8,9 | 0,43 ($CHCl_3$) |
| 12 | C 60,0 | H 9,3 | C 60,1 | H 9,2 | 0,58 ($CH_2Cl_2$:Heptan:Methanol = 16:16:1) |
| 13 | C 55,8 | H 7,0 | C 55,7 | H 6,9 | 0,35 ($CHCl_3$) |
| 14 | C 62,9 | H 6,7 | C 62,7 | H 6,7 | 0,77 ($CHCl_3$:Hexan:Methanol = 8:8:1) |
| 15 | C 55,0 | H 8,3 | C 55,2 | H 8,2 | 0,31 ($CHCl_3$:Hexan:Methanol = 4:4:1) |
| 16 | C 59,0 | H 8,3 | C 59,1 | H 8,4 | 0,57 ($CH_2Cl_2$:Ethylacetat = 4:1) |
| 17 | C 60,5 | H 8,6 | C 60,2 | H 8,6 | 0,47 ($CHCl_3$:Methanol:Hexan = 4:1:4) |
| 18 | C 67,1 | H 6,6 | C 67,0 | H 6,5 | 0,43 ($CHCl_3$:Hexan:Methanol = 4:4:1) |
| 19 | C 55,8 | H 7,0 | C 55,9 | H 7,1 | 0,63 ($CHCl_3$:Hexan:Methanol = 8:8:1) |
| 20 | C 67,9 | H 5,7 | C 68,1 | H 5,9 | 0,79 ($CHCl_3$:Hexan:Methanol = 8:8:1) |
| 21 | C 59,4 | H 7,3 | C 59,6 | H 7,2 | 0,45 ($CHCl_3$:Hexan:Methanol = 16:16:1) |
| 22 | C 63,7 | H 6,3 | C 63,7 | H 6,2 | 0,54 ($CHCl_3$:Hexan:Methanol = 8:8:1) |
| 23 | C 64,0 | H 9,4 | C 64,1 | H 9,6 | 0,72 ($CH_2Cl_2$:Ethylacetat = 4:1) |
| 24 | C 56,6 | H 5,7 | C 56,8 | H 5,7 | 0,35 (Ethylacetat:Methanol = 19:1) |

Beispiel 9

Monolayer von HEP-G2-Zellen in lipoproteinfreiem Nährmedium werden mit entsprechenden Konzentrationen der zu prüfenden Substanzen aus den Beispielen 1, 3, 4, 5, 6, 8 und 15 eine Stunde vorinkubiert. Nach Zugabe der $^{14}C$-markierten Biosynthesevorstufe [$^{14}C$]Natriumacetat wird die Inkubation für 3 Stunden fortgesetzt. Danach wird ein Teil der Zellen alkalisch verseift, bei vorheriger Zugabe eines internen Standards von $^{3}H$-Cholesterin. Die Lipide der verseiften Zellen werden mit einem Gemisch aus Chloroform-Methanol extrahiert. Dieses Lipidgemisch wird nach Zusatz von Trägercholesterin präparativ dünnschichtchromatographisch aufgetrennt, die Cholesterinbande nach Anfärbung isoliert und die aus dem $^{14}C$-Precursor gebildete Menge $^{14}C$-Cholesterin szintigraphisch bestimmt. In einem aliquoten Teil der Zellen wurde Zellprotein bestimmt, sodaß die in der Zelleinheit pro mg Zellprotein aus $^{14}C$-Vorläufer gebildete Menge $^{14}C$-Cholesterin berechnet werden kann. Zum Vergleich für die Hemmwirkung eines zugesetzten Prüfpräparates dient die Kontrolle, so daß direkt die Hemmung der Cholesterinbiosynthese bei einer bestimmten molaren Konzentration des Prüfpräparates im Medium angegeben werden kann. In aliquoten Anteilen der Zellkultur wird die Intaktheit der Zellkultur und die fehlende Zellschädigung durch Präparateeinwirkung morphologisch (Lichtmikroskopie) beurteilt und biochemisch durch Bestimmung der Laktat-Dehydrogenase Ausschüttung im Inkubationsmedium gemessen. Als Standardpräparat wurde ®Lovastatin (Merck-Sharp-Dohm) benutzt. Die Ergebnisse sind in Tabelle 3 wiedergegeben.

Tabelle 3

| Verbindung | Konzentration [M] | Cholesterin in Prozent der Kontrolle |
|---|---|---|
| 1 | $10^{-8}$ | 41 |
| 3 | $10^{-6}$ | 28 |
| 3 | $10^{-8}$ | 33 |
| 4 | $10^{-8}$ | 45 |
| 5 | $10^{-8}$ | 33 |
| 6 | $10^{-5}$ | 32 |
| 6 | $10^{-7}$ | 30 |
| 8 | $10^{-8}$ | 67 |
| 15 | $10^{-8}$ | 73 |
| Lovastatin | $10^{-8}$ | 55 |

Antivirale Wirksamkeit in der Zellkultur

Die Prüfsubstanzen wurden in Zellkulturmedium (Dulbecco's MEM) gelöst und in einer geometrischen Verdünnungsreihe, Faktor 3, in Standard-Mikrotiterplatten in 100 µl Zellkulturmedium vorgelegt. Anschließend erfolgte die Zugabe von 100 µl einer Suspension von HeLa- bzw. Vero-Zellen in Medium mit 5 % fötalem Kälberserum in einer Zelldichte von 2 x $10^5$ Zellen/ml. Die Ansätze wurden mit 50 µl einer Suspension des jeweiligen Test-Virus infiziert, die so eingestellt war, daß die Zellen innerhalb von 72 h einen cytopathogenen Effekt (CPE) zeigten. Die Auswertung erfolgte durch mikroskopische Begutachtung des Zellrasens und photometrische Messung der Neutralrotaufnahme (Farbtest nach Finter). Als MHK wurde die Konzentration des Präparats angenommen (µg/ml), bei der etwa 50 % der Zellen die Infektion überlebten.

In Tabelle 4 ist die Wirkung (Angabe des MHK in µg/ml) von der erfindungsgemäßen Verbindung 6 gegen folgende Viren aufgeführt:
Adeno 5, Vaccina, Herpes I, Herpes II, Influenza A, Paramyxo. III, Rhinovirus II. In der letzten Spalte ist die DTM (Dosis Tolerate Maxima) in µg/ml angegeben.

Tabelle 4

| MHK in µg/ml | | | | | | | |
|---|---|---|---|---|---|---|---|
| Verb. aus Bsp. | Adeno 5 | Vaccina | Herpes I | Herpes II | Influenza A | Rhino. II | DTM [µg/ml] |
| 6 | 0,55 | 0,49 | 0,55 | 0,55 | 14,0 | 44,4 | 133,3 |

## Patentansprüche

**1.** Decarestrictine der Formel I

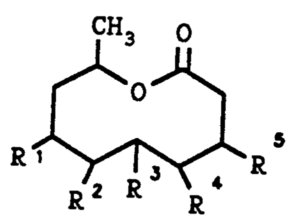

(I)

wobei $R^1$ für
a) Hydroxy,

b) Formyl,
c) Carbonyl,
d) -O-($C_1$-$C_{10}$)-Alkyl, worin die Kohlenstoffkette geradkettig oder verzweigt sein kann,
e) O-($C_2$-$C_{10}$)-Alkenyl, worin die Kohlenstoffkette geradkettig oder verzweigt sein kann und eine oder mehrere Doppelbindungen enthält,
f) -O-C(O)-($C_1$-$C_{10}$)-Alkyl, worin die Kohlenstoffkette geradkettig oder verzweigt sein kann,
g) -O-C(O)-($C_2$-$C_{10}$)-Alkenyl, worin die Kohlenstoffkette geradkettig oder verzweigt sein kann und eine oder mehrere Doppelbindungen enthält,
h) -O-C(O)-Phenyl,
i) -O-C(O)-Phenyl, ein- bis dreifach substituiert am Phenyl-Rest durch
1) Halogen, wie Fluor, Chlor oder Brom,
2) ($C_1$-$C_8$)-Alkyl, worin die Kohlenstoffkette geradkettig oder verzweigt sein kann,
3) $NO_2$,
4) CN,
5) $CF_3$,
6) Phenyl oder
7) Phenoxy,
j) Mono- oder Disaccharid oder
k) Mono- oder Disaccharid, deren Hydroxylgruppen mit Schutzgruppen geschützt sind, steht,

$R^2$ und $R^3$ für Wasserstoff stehen oder
$R^2$ und $R^3$ zusammen eine Doppelbindung oder einen Oxiranring bilden,
$R^4$ oder $R^5$ unabhängig voneinander für
a) Hydroxy,
b) Carbonyl,
c) -O-($C_1$-$C_{10}$)-Alkyl, worin die Kohlenstoffkette geradkettig oder verzweigt sein kann,
d) -O-($C_2$-$C_{10}$)-Alkenyl, worin die Kohlenstoffkette geradkettig oder verzweigt sein kann und eine oder mehrere Doppelbindungen enthält.
e) -O-C(O)-($C_1$-$C_{10}$)-Alkyl, worin die Kohlenstoffkette geradkettig oder verzweigt sein kann,
f) -O-C(O)-($C_2$-$C_{10}$)-Alkenyl, worin die Kohlenstoffkette geradkettig oder verzweigt sein kann und eine oder mehrere Doppelbindungen enthält,
g) -O-C(O)-Phenyl oder
h) -O-C(O)-Phenyl, ein- bis dreifach substituiert am Phenyl-Rest durch
1) Halogen, wie Fluor, Chlor oder Brom,
2) ($C_1$-$C_8$)-Alkyl, worin die Kohlenstoffkette geradkettig oder verzweigt sein kann,
3) $NO_2$,
4) CN,
5) $CF_3$,
6) Phenyl oder
7) Phenoxy steht, oder

$R^4$ und $R^5$ zusammen einen Rest der Formel II oder III

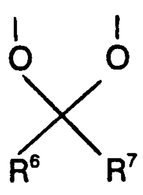 (II)

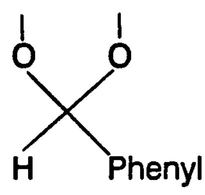 (III)

bilden, wobei $R^6$ oder $R^7$ unabhängig voneinander
a) Wasserstoff oder
b) ($C_1$-$C_8$)-Alkyl, worin die Kohlenstoffkette geradkettig oder verzweigt sein kann, bedeuten,

sowie deren physiologisch verträglichen Salze,
ausgenommen die Verbindung der Formel I, wobei $R^1$ und $R^4$ für Hydroxylgruppe, $R^5$ für Hydroxyl- oder Carbonylgruppe steht und $R^2$ und $R^3$ zusammen eine Doppelbindung bilden.

**2.** Decarestrictine der Formel I nach Anspruch I,
wobei $R^1$ für

a) Hydroxy,
b) Formyl,
c) Carbonyl,
d) -O-($C_1$-$C_4$)-Alkyl,
e) -O-C(O)-($C_1$-$C_4$)-Alkyl, worin die Kohlensotffkette geradkettig oder verzweigt sein kann,
f) -O-C(O)-($C_2$-$C_4$)-Alkenyl, worin die Kohlenstoffkette geradkettig oder verzweigt sein kann,
g) -O-C(O)-Phenyl,
h) -O-C(O)-Phenyl, ein- bis dreifach substituiert am Phenyl-Rest durch Brom, steht oder
i) Monosaccharid, dessen Hydroxylgruppen durch Benzoyl geschützt sind,

$R^2$ und $R^3$ für Wasserstoff stehen oder
$R^2$ und $R^3$ zusammen eine Doppelbindung oder einen Oxiranring bilden,
$R^4$ oder $R^5$ unabhängig voneinander

a) Hydroxy,
b) Carbonyl,
c) -O-($C_1$-$C_4$)-Alkyl,
d) -O-C(O)-($C_1$-$C_4$)-Alkyl, worin die Kohlenstoffkette geradkettig oder verzweigt sein kann,
e) -O-C(O)-($C_2$-$C_4$)-Alkenyl, worin die Kohlenstoffkette geradkettig oder verzweigt sein kann, oder
f) -O-C(O)-Phenyl bedeuten oder

$R^4$ und $R^5$ zusammen einen Rest der Formel II oder III bilden, wobei $R^6$ oder $R^7$ für jeweils eine Methylgruppe steht, sowie deren physiologisch verträglichen Salze, ausgenommen die Verbindungen der Formel I, wobei $R^1$ und $R^4$ für Hydroxylgruppe, $R^5$ für Hydroxyl- oder Carbonylgruppe steht und $R^2$ und $R^3$ zusammen eine Doppelbindung bilden.

**3.** Verfahren zur Herstellung von Decarestrictine der Formel I nach Anspruch 1, dadurch gekennzeichnet, daß man

a) die Verbindung der Formel Ia

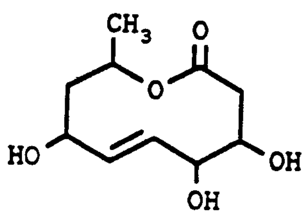

(Ia)

umsetzt mit einer Verbindung der Formel IV

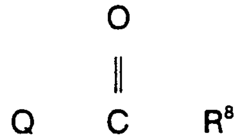

(IV)

wobei Q für

1) Hydroxy,
2) Halogen, wie Chlor oder Brom,
3) Imidazolid oder
4) Säureanhydrid steht,

$R^8$ für eine Alkylgruppe mit 1 bis 10 C-Atomen oder eine Alkenylgruppe mit 2 bis 10 C-Atomen steht,

oder daß man

b) die Verbindung der Formel Ia mit einem geeigneten Enzym und einem Benzoyl- oder Acyldonor umsetzt,

oder daß man

c) die Verbindung der Formel Ia zu einer Verbindung der Formel V oxidiert,

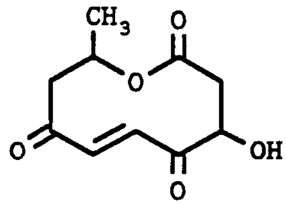

(V)

oder daß man

d) die Verbindung der Formel Ia an der Doppelbindung zu einer Verbindung der Formel VI epoxidiert,

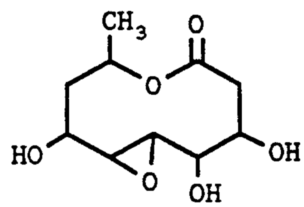

(VI)

oder daß man

e) die Verbindung der Formel Ia an der Doppelbindung zu einer Verbindung der Formel VII hydriert,

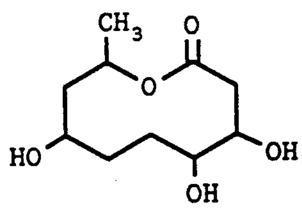

(VII)

oder daß man

f) die Verbindung der Formel Ia mit einer Verbindung der Formel

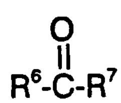

oder Benzaldehyd in das entsprechende Ketal der Formel VIII oder Benzylidenketal der Formel IX überführt,

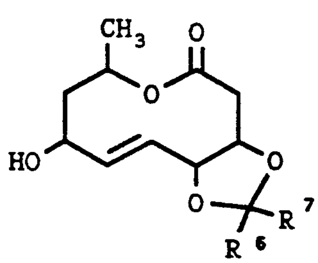

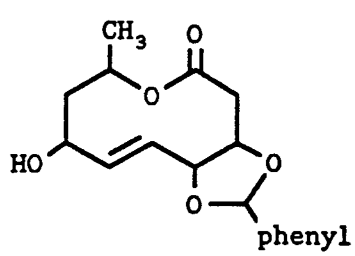

(VIII) (IX)

wobei $R^6$ oder $R^7$ unabhängig voneinander

1) Wasserstoff oder

2) ($C_1$-$C_8$)-Alkyl, worin die Kohlenstoffkette geradkettig oder verzweigt sein kann, bedeutet,

oder daß man

g) die Verbindung der Formel VIII oder IX an der Hydroxygruppe mit einer Verbindung der Formel X,

$R^{10}$-Hal (X)

wobei $R^{10}$ Mono- oder Disaccharid bedeutet und Hal für Fluor, Chlor, Brom oder Jod steht, umsetzt,

oder daß man

h) die Verbindung der Formel Ia mit einer Verbindung der Formel XI

$R^9$-Abg (XI)

umsetzt, wobei $R^9$ für

1) ($C_1$-$C_{10}$)-Alkyl, worin die Kohlenstoffkette geradkettig oder verzweigt sein kann, oder

2) ($C_2$-$C_{10}$)-Alkenyl, worin die Kohlenstoffkette geradkettig oder verzweigt sein kann und eine oder mehrere Doppelbindungen enthält, steht und

Abg Chlor, Brom, Jod, Sulfat, Mesylat oder Tosylat bedeutet,

oder daß man

zwei oder mehrere der obengenannten Verfahrensvarianten a) bis h) hintereinander ausführt oder daß man anschließend die Verbindung der Formel I in ihre physiologisch verträglichen Salze überführt.

**4.** Arzneimittel, enthaltend eine wirksame Menge mindestens einer Verbindung der Formel I gemäß Anspruch 1 oder 2 oder wie erhalten nach Anspruch 3 und/oder mindestens eines physiologisch verträglichen Salzes einer solchen Verbindung neben einem physiologisch annehmbaren Träger und gegebenenfalls weiteren Zustz- und/oder Hilfsstoffen.

**5.** Verfahren zur Herstellung eines Arzneimittels gemäß Anspruch 4, dadurch gekennzeichnet, daß man mindestens eine Verbindung der Formel I und/oder mindestens ein physiologisch verträgliches Salz einer solchen Verbindung und/oder eine nach dem Verfahren gemäß Anspruch 3 erhaltene Verbindung mit einem physiologisch annehmbaren Träger und gegebenenfalls weiteren Zusatz- und/oder Hilfsstoffen in eine geeignete Darreichungsform bringt.

**6.** Verwendung der Verbindung der Formel I nach Anspruch 1 oder 2 zur Herstellung eines Arzneimittels mit lipidsenkender Wirkung.

**7.** Verwendung der Verbindung der Formel I nach Anspruch 1 oder 2 zur Herstellung eines Arzneimittels mit antiviraler Wirkung.

**Patentansprüche für folgende Vertragsstaaten : ES, GR**

**1.** Verfahren zur Herstellung von Decarestrictinen der Formel I

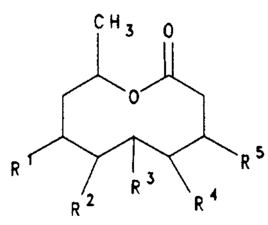

(I)

wobei $R^1$ für

a) Hydroxy,

b) Formyl,

c) Carbonyl,

d) -O-($C_1$-$C_{10}$)-Alkyl, worin die Kohlenstoffkette geradkettig oder verzweigt sein kann,
e) -O-($C_2$-$C_{10}$)-Alkenyl, worin die Kohlenstoffkette geradkettig oder verzweigt sein kann und eine oder mehrere Doppelbindungen enthält,
f) -O-C(O)-($C_1$-$C_{10}$)-Alkyl, worin die Kohlenstoffkette geradkettig oder verzweigt sein kann,
g) -O-C(O)-($C_2$-$C_{10}$)-Alkenyl, worin die Kohlenstoffkette geradkettig oder verzweigt sein kann und eine oder mehrere Doppelbindungen enthält,
h) -O-C(O)-Phenyl,
i) -O-C(O)-Phenyl, ein- bis dreifach substituiert am Phenyl-Rest durch
1) Halogen, wie Fluor, Chlor oder Brom,
2) ($C_1$-$C_8$)-Alkyl, worin die Kohlenstoffkette geradkettig oder verzweigt sein kann,
3) $NO_2$,
4) CN,
5) $CF_3$,
6) Phenyl oder
7) Phenoxy,
j) Mono- oder Disaccharid oder
k) Mono- oder Disaccharid, deren Hydroxylgruppen mit Schutzgruppen geschützt sind, steht,
$R^2$ und $R^3$ für Wasserstoff stehen oder
$R^2$ und $R^3$ zusammen eine Doppelbindung oder einen Oxiranring bilden,
$R^4$ oder $R^5$ unabhängig voneinander für
a) Hydroxy,
b) Carbonyl,
c) -O-($C_1$-$C_{10}$)-Alkyl, worin die Kohlenstoffkette geradkettig oder verzweigt sein kann,
d) -O-($C_2$-$C_{10}$)-Alkenyl, worin die Kohlenstoffkette geradkettig oder verzweigt sein kann und eine oder mehrere Doppelbindungen enthält.
e) -O-C(O)-($C_1$-$C_{10}$)-Alkyl, worin die Kohlenstoffkette geradkettig oder verzweigt sein kann,
f) -O-C(O)-($C_2$-$C_{10}$)-Alkenyl, worin die Kohlenstoffkette geradkettig oder verzweigt sein kann und eine oder mehrere Doppelbindungen enthält,
g) -O-C(O)-Phenyl oder
h) -O-C(O)-Phenyl, ein- bis dreifach substituiert am Phenyl-Rest durch
1) Halogen, wie Fluor, Chlor oder Brom,
2) ($C_1$-$C_8$)-Alkyl, worin die Kohlenstoffkette geradkettig oder verzweigt sein kann,
3) $NO_2$,
4) CN,
5) $CF_3$,
6) Phenyl oder
7) Phenoxy steht, oder
$R^4$ und $R^5$ zusammen einen Rest der Formel II oder III

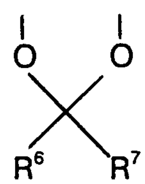 (II)

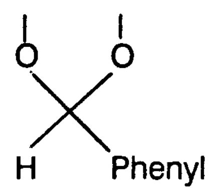 (III)

bilden, wobei $R^6$ oder $R^7$ unabhängig voneinander
a) Wasserstoff oder
b) ($C_1$-$C_8$)-Alkyl, worin die Kohlenstoffkette geradkettig oder verzweigt sein kann, bedeuten,
sowie deren physiologisch verträglichen Salze,
ausgenommen die Verbindung der Formel I, wobei $R^1$ und $R^4$ für Hydroxylgruppe, $R^5$ für Hydroxyl- oder Carbonylgruppe steht und $R^2$ und $R^3$ zusammen eine Doppelbindung bilden, dadurch gekennzeichnet, daß man
a] die Verbindung der Formel Ia

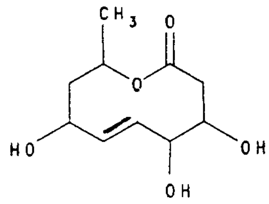

(Ia)

umsetzt mit einer Verbindung der Formel IV

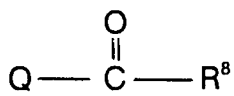

(IV)

wobei Q für

1) Hydroxy,
2) Halogen, wie Chlor oder Brom,
3) Imidazolid oder
4) Säureanhydrid steht,

$R^8$ für eine Alkyl- oder Alkenylgruppe mit 1 bis 10 C-Atomen steht, oder daß man

b) die Verbindung der Formel Ia mit einem geeigneten Enzym und einem Benzyl- oder Acyldonor umsetzt,

oder daß man

c) die Verbindung der Formel Ia zu einer Verbindung der Formel V oxidiert,

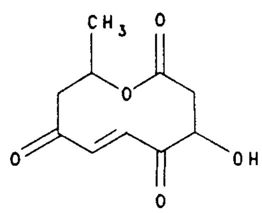

(V)

oder daß man

d) die Verbindung der Formel Ia an der Doppelbindung zu einer Verbindung der Formel VI epoxidiert,

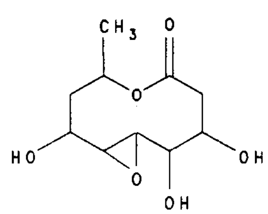

(VI)

oder daß man

e) die Verbindung der Formel Ia an der Doppelbindung zu einer Verbindung der Formel VII hydriert,

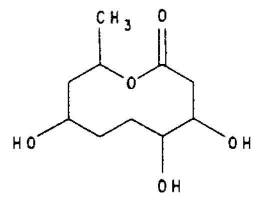

(VII)

oder daß man

f) die Verbindung der Formel Ia mit einer Verbindung der Formel

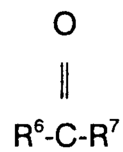

oder Benzaldehyd in das entsprechende Ketal der Formel VIII oder Benzylidenketal der Formel IX überführt,

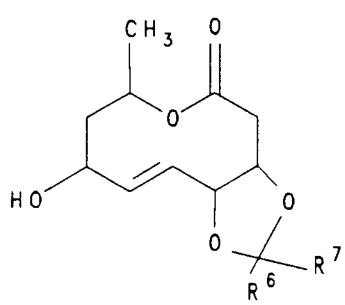

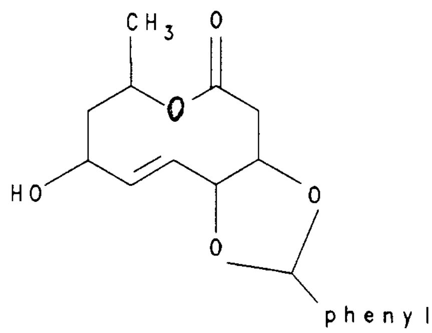

(VIII) (IX)

wobei $R^6$ oder $R^7$ unabhängig voneinander

1) Wasserstoff oder

2) ($C_1$-$C_8$)-Alkyl, worin die Kohlenstoffkette geradkettig oder verzweigt sein kann, bedeutet,

oder daß man

g) die Verbindung der Formel VIII oder IX an der Hydroxygruppe mit einer Verbindung der Formel X,

$R^{10}$-Hal (X)

wobei $R^{10}$ Mono- oder Disaccharid bedeutet und Hal für Fluor, Chlor, Brom oder Jod steht, umsetzt,

oder daß man

h) die Verbindung der Formel Ia mit einer Verbindung der Formel XI

$R^9$-Abg (XI)

umsetzt, wobei $R^9$ für

1) ($C_1$-$C_{10}$)-Alkyl, worin die Kohlenstoffkette geradkettig oder verzweigt sein kann, oder

2) ($C_2$-$C_{10}$)-Alkenyl, worin die Kohlenstoffkette geradkettig oder verzweigt sein kann und eine oder mehrere Doppelbindungen enthält, steht und

Abg Chlor, Brom, Jod, Sulfat, Mesylat oder Tosylat bedeutet,

oder daß man

zwei oder mehrere der obengenannten Verfahrensvarianten a) bis h) hintereinander ausführt oder daß man anschließend die Verbindung der Formel I in ihre physiologisch verträglichen Salze überführt.

2. Verfahren nach Anspruch 1, dadurch gekennzeichnet, daß Decar estrictine der Formel I nach Anspruch 1 hergestellt werden,
wobei $R^1$ für
a) Hydroxy,
b) Formyl,
c) Carbonyl,
d) -O-($C_1$-$C_4$)-Alkyl,
e) -O-C(O)-($C_1$-$C_4$)-Alkyl, worin die Kohlensotffkette geradkettig oder verzweigt sein kann,
f) -O-C(O)-($C_2$-$C_4$)-Alkenyl, worin die Kohlenstoffkette geradkettig oder verzweigt sein kann,
g) -O-C(O)-Phenyl,
h) -O-C(O)-Phenyl, ein- bis dreifach substituiert am Phenyl-Rest durch Brom, steht oder
i) Monosaccharid, dessen Hydroxylgruppen durch Benzoyl geschützt sind,
$R^2$ und $R^3$ für Wasserstoff stehen oder
$R^2$ und $R^3$ zusammen eine Doppelbindung oder einen Oxiranring bilden,
$R^4$ oder $R^5$ unabhängig voneinander
a) Hydroxy,
b) Carbonyl,
c) -O-($C_1$-$C_4$)-Alkyl,
d) -O-C(O)-($C_1$-$C_4$)-Alkyl, worin die Kohlenstoffkette geradkettig oder verzweigt sein kann,
e) -O-C(O)-($C_2$-$C_4$)-Alkenyl, worin die Kohlenstoffkette geradkettig oder verzweigt sein kann, oder
f) -O-C(O)-Phenyl bedeuten oder
$R^4$ und $R^5$ zusammen einen Rest der Formel II oder III bilden, wobei $R^6$ oder $R^7$ für jeweils eine Methylgruppe steht, sowie deren physiologisch verträglichen Salze, ausgenommen die Verbindungen der Formel I, wobei $R^1$ und $R^4$ für Hydroxylgruppe, $R^5$ für Hydroxyl- oder Carbonylgruppe steht und $R^2$ und $R^3$ zusammen eine Doppelbindung bilden.

3. Arzneimittel, enthaltend eine wirksame Menge mindestens einer Verbindung der Formel I gemäß Anspruch 1 oder 2 und/oder mindestens eines physiologisch verträglichen Salzes einer solchen Verbindung neben einem physiologisch annehmbaren Träger und gegebenenfalls weiteren Zustz- und/oder Hilfsstoffen.

4. Verfahren zur Herstellung eines Arzneimittels gemäß den Ansprüchen 1 oder 2, dadurch gekennzeichnet, daß man mindestens eine Verbindung der Formel I und/oder mindestens ein physiologisch verträgliches Salz einer solchen Verbindung mit einem physiologisch annehmbaren Träger und gegebenenfalls weiteren Zusatz- und/oder Hilfsstoffen in eine geeignete Darreichungsform bringt.

5. Verwendung einer Verbindung der Formel I nach den Ansprüchen 1 oder 2 zur Herstellung eines Arzneimittels mit lipidsenkender Wirkung.

6. Verwendung einer Verbindung der Formel I nach den Ansprüchen 1 oder 2 zur Herstellung eines Arzneimittels mit antiviraler Wirkung.

European Patent Office

# EUROPEAN SEARCH REPORT

Application Number

EP 92 10 8785

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,A | EP-A-0 333 024 (HOECHST) * page 2 * ----- | 1,4-7 | C07D313/00 A61K31/365 C07D493/04 C07H17/08 C12P17/08 //(C07D493/04, 313:00,303:00) (C07D493/04, 317:00,313:00) |
| | | | TECHNICAL FIELDS SEARCHED (Int. Cl.5 ) |
| | | | C07D |

The present search report has been drawn up for all claims

| Place of search | Date of completion of the search | Examiner |
|---|---|---|
| THE HAGUE | 09 JULY 1992 | RUSSELL F. ENGLISH |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)